# EUROPEAN PATENT APPLICATION

(11) **EP 4 682 137 A1**
(43) Date of publication of application: **21.01.2026**
(21) Application number: 24189184.5
(22) Date of filing: 17.07.2024
(51) Int. Cl.: C07C 201/16, C07C 205/06, C07C 209/86, C07C 211/47

(54) **EFFICIENT ENERGY USE IN CHEMICAL PROCESSES**

(71) Applicant: BASF SE, 67056 Ludwigshafen am Rhein (DE)
(72) Inventor: MATTERN, Jonas, 01987 Schwarzheide (DE); BECK, Benjamin, 01987 Schwarzheide (DE); SCHOLZ, Mathias, 01987 Schwarzheide (DE); WALLROTH-SCHODER, Andre, 01987 Schwarzheide (DE); PREUSCHE, Robert, 01987 Schwarzheide (DE)
(74) Representative: BASF IP Association

(57) **Abstract**

The present disclosure relates to methods, apparatuses, systems and computer elements for monitoring and/or controlling energy consumption, monitoring and/or controlling emissions related to energy consumption and/or operating chemical processes in an energy and/or cost-efficient manner.

## Description

### TECHNICAL FIELD

The present disclosure relates to methods, apparatuses, processes, systems, uses and computer elements for producing aromatic amins and/or aromatic nitro chemicals by monitoring and/or controlling at least one thermal separation step based on energy consumption data.

### TECHNICAL BACKGROUND

Industrial processes have high energy demands. There are unsteady electric energy availabilities with respect to type and price due to a rising number of renewable energies in the general energy mix. These variabilities are caused by external influences like weather conditions and cannot be influenced. Commonly, the power plant management and the corresponding power plant (or power plants) recognize these and optimize the energy production with focus on minimizing the costs and/ or maximizing the profit. In the chemical industry it is common that power plants not only supply the receiving users with electric energy but also with steam and/ or other energies. There is a need to use the energy with highest efficiency.

### SUMMARY OF THE INVENTION

In one aspect disclosed is a method for monitoring and/or controlling at least one thermal separation step in the production of aromatic amins and/or aromatic nitro chemicals, the method comprising the steps of:
- providing energy data associated with input energy supply characteristics and/or consumption characteristics,
- determining based on the energy data input data associated with one or more input mass stream(s) to be fed to the at least one thermal separation step,
- monitoring and/or controlling, based on the input data, the one or more input mass stream(s) fed to the at least one thermal separation step.

In another aspect disclosed is an apparatus for monitoring and/or controlling at least one thermal separation step in the production of aromatic amins and/or aromatic nitro chemicals, the apparatus configured to:
- providing energy data associated with input energy supply characteristics and/or consumption characteristics,
- determining based on the energy data input data associated with one or more input mass stream(s) to be fed to the at least one thermal separation step,
- monitoring and/or controlling, based on the input data, the one or more input mass stream(s) fed to the at least one thermal separation step.

Method for producing aromatic amins and aromatic nitro chemicals, the method comprising at least one thermal separation step, wherein the thermal separation step is monitored and/or controlled by the methods or apparatuses disclosed herein.

In another aspect disclosed is an industrial process for producing aromatic amins and aromatic nitro chemicals, operated according to the methods or by the apparatuses disclosed herein.

In another aspect disclosed is a chemical product produced using an industrial process for producing aromatic amins and aromatic nitro chemicals, operated according to the methods or by the apparatuses disclosed herein.

In another aspect disclosed is an use of the operational instructions generated according to any of the methods or by the apparatus disclosed herein for operating one or more industrial process(es) with one or more operation units.

In another aspect disclosed is an use of the input data generated according to any of methods disclosed herein for operating an industrial process.

### EMBODIMENTS

For primarily electric energy consuming plants like electrolysis plants (e.g. hydrogen, chloralkali process, ...) it is common that energy usage is optimized. For other plants like distillation, rectification-, extraction-, crystallization-plants or other plants it is not common that energy costs are a part of the short-term production management. The power plant cost/ profit optimization system does not include the named downstream plants. Due to changing electric energy prices every minute, there is a significant profit maximization potential if the downstream plants are included. If an industrial chemical plant has the ability to react to fast changing energy prices, the plant can be a part of the cost/profit optimization system. In the specific case a plant for the thermal separation of nitrotoluenes and a plant for the thermal separation of aromatic amines was updated with respect to on line analytics to perform changes in the amount of feed in less than a few minutes without exceeding critical limits (like quality or others). Power plants can produce steam and electric energy simultaneously. This is caused by the functional principle of the power plant, which is a common technology. Due to economic factors or other circumstances (e.g. weather) it could be lucrative to produce much (or less) electric energy. The energy (steam) consumption of the downstream plants may be fitted to the current electric energy production. The energy (steam) consumption of the downstream plants can be modified by modifying the feed of the plants. It is possible that the electric energy demand is changing multiple times a day such as short term like every 15 minutes. Therefore, the feed of the downstream plants is accommodated multiple times a day too. By including the downstream plants in the energy cost/ profit optimization, a significant profit is achievable.

In one embodiment the energy data relates to energy type and/or energy mix.

In another embodiment the energy type relates to solar-, wind-, gas- or fossil-based energy, wherein the energy mix may relate to a mix of multiple energy types.

In another embodiment energy data may relate to time dependent behavior of input energy supply characteristics and/or consumption characteristics.

In another embodiment the energy data relates to current input energy supply characteristics and/or consumption characteristics.

In another embodiment input data is determined based on current input energy supply characteristics and/or consumption characteristics.

In another embodiment determining input data includes determining quantity of input feed and/or heat input to be fed to the at least one thermal separation step.

In another embodiment the input data is provided to a input feed for monitoring and/or controlling one or more input mass stream(s) fed to the at least one thermal separation step.

In another embodiment depending on fluctuations in the energy data, different feed streams to at least one thermal separation step are adapted.

In another embodiment the at least one thermal separation step includes a sequential column setup configured to separate ortho, -meta, para- nitrotoluol isomers.

In another embodiment the at least one thermal separation step includes a sequential column setup configured to separate a toluidine mixture.

### BRIEF DESCRIPTION OF DRAWINGS

In the following, the present disclosure is further described with reference to the enclosed figures:
- Fig. 1: illustrates an example of a chemical process setup including upstream and downstream processes.
- Fig. 2: illustrates an example of a method for monitoring and/or controlling at least one thermal separation step in the production of aromatic amins and/or aromatic nitro chemicals.
- Fig. 3: illustrates an example of an apparatus for monitoring and/or controlling at least one thermal separation step in the production of aromatic amins and/or aromatic nitro chemicals.

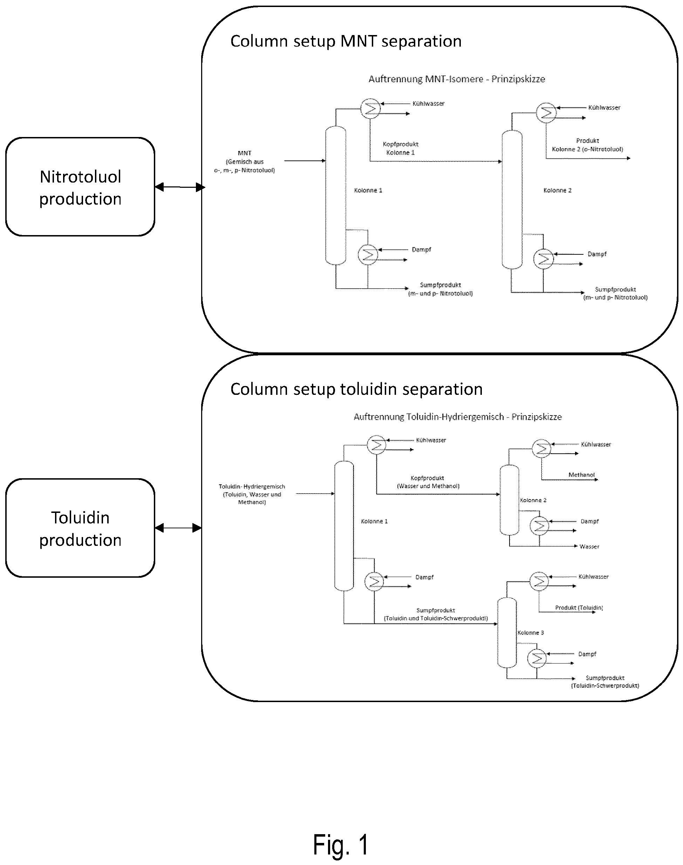

Fig. 1 illustrates an example of a chemical process setup including upstream and downstream processes.

The chemical process setup may comprise one or more process steps converting raw material educts to one or more products. At least one process steps may comprise at least one chemical reaction for producing the one or more products. For example, the process setup may be configured to produce aromatic nitro and/or amino compounds such as toluidine. A nitration step may convert toluol to nitro toluol compounds. A hydrogenation step may convert nitro toluol compounds to toluidine. Thermal separation steps may separate ortho, meta and/or para substituents.

Chemical processes consume electric energy and are often operated in a cost / profit optimized way. In particular up stream plants like electrolysis plants including e.g. hydrogen, chloralkali process or the like exhibit higher electric energy demand than more downstream plants like distillation-, rectification-, extraction-, crystallization- plants or other plants. In addition the down stream plants with their energy consumption can be used for short term energy management of chemical process setup such as the one illustrated in Fig. 1. Due to changing electric energy characteristics every minute, there is a significant profit maximization potential if the downstream plants are considered. If an industrial chemical plant can react to fast changing energy characteristics, the plant can be a part of the cost/ profit optimization system. In the example illustrated here, a plant for the thermal separation of nitrotoluenes and a plant for the thermal separation of aromatic amines may be monitored and/or controlled with respect to the amount of feed in view of the available energy characteristics in less than a few minutes without impeding operational limits such as quality or yield.

Example separation columns are illustrated in Fig. 1. One may separate ortho, -meta, para- nitrotoluol isomers via a sequential column setup with two columns. Another may separate toluidine mixture via a sequential column setup with three columns. The feeds to the column setup or to individual columns may be monitored and/or controlled in accordance with the energy available to operate the column setups. Such monitoring and/or controlling allows for short-term adaption taking variations in the energy supply and/or consumption characteristics into account. To enhance the flexibility the feeds to the column setup or to individual columns may include regulated feed valves configured to regulate the feed volume. In addition buffer volumes may be included which may allow to buffer feed stream.

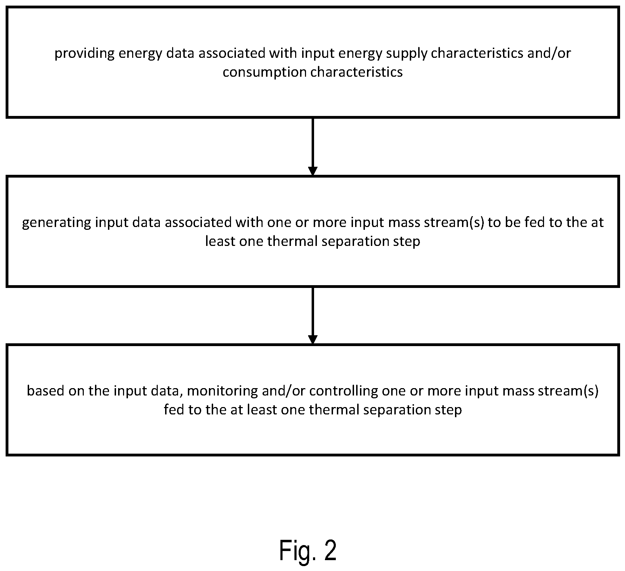

Fig. 2 illustrates an example of a method for monitoring and/or controlling at least one thermal separation step in the production of aromatic amins and/or aromatic nitro chemicals.

Energy data associated with input energy supply characteristics and/or consumption characteristics may be provided. The supply characteristics may relate to the energy type and/or energy mix. For example, the energy type may relate to solar-, wind-, gas- or fossil-based energy. Further for example, the energy mix may relate to a mix of multiple energy types. The energy type and/or mix may specify the relative portion(s) of individual energy types, and/or the energy price point per energy type. The consumption characteristics may relate to the usage of the energy such as per energy type or mix.

The energy data may relate to time series data associated with input energy supply characteristics and/or consumption characteristics. The energy data may relate to time dependent behavior of input energy supply characteristics and/or consumption characteristics. The energy data may relate to current, time dependent behavior of input energy supply characteristics and/or consumption characteristics. The energy data may relate to time dependent behavior of input energy supply characteristics and/or consumption characteristics on minute to hour scale.

Based on the energy data, input data associated with one or more input mass stream(s) to be fed to the at least one thermal separation step may be determined. The input data may be determined based on current input energy supply characteristics and/or consumption characteristics. The input data may be associated with one or more input mass stream(s) to be fed to at least one thermal separation step.

Based on the input data, the one or more input mass stream(s) fed to the at least one thermal separation step may be monitored and/or controlled.

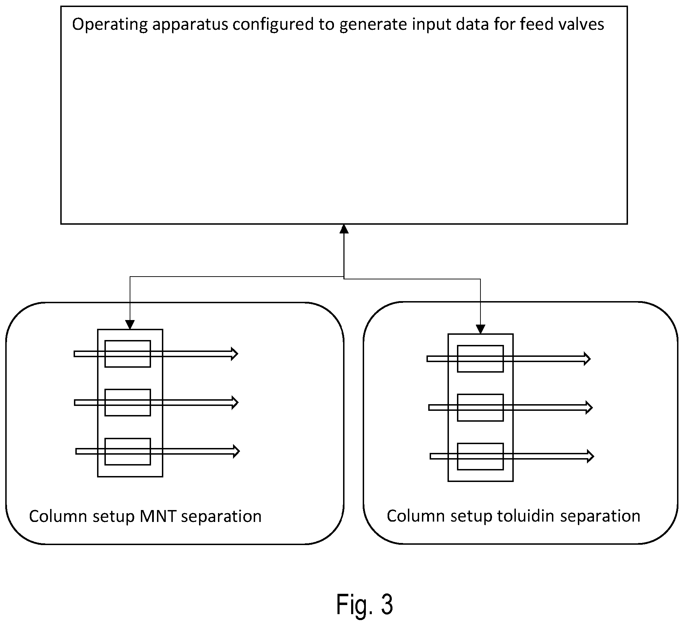

Fig. 3 illustrates an example of an apparatus for monitoring and/or controlling at least one thermal separation step in the production of aromatic amins and/or aromatic nitro chemicals.

An operating apparatus may be configured for controlling the separation steps as for example illustrated in the context of Fig. 1. The operating apparatus may be configured to receive energy data, determine input data and provide the input data for monitoring and/or controlling as e.g. described in the context of Fig. 2. The operating apparatus may be communicatively coupled to monitoring and/or controlling equipment of the separation steps. The separation steps may be monitored and/or controlled by actuators such as feed inlet valves or sensors such as flow sensors or temperature sensors.

The operating apparatus may be configured to determine the input data associated with one or more input mass stream(s) to be fed to one or more columns or column setups. The input data may for example be generated based on the column setup and the feed streams to the column setup of the individual columns and the energy data. Depending on the energy data fluctuations, the different feed streams to the column setups or individual columns can be adapted accordingly. For example, if low fraction of renewable energy is available according to the energy data, the feed streams to the column setup of the individual columns can be reduced. This way the energy consumption of the column setup can be adapted to the externally available energy. Such control can even be operated online achieving near real time controlling of input feed stream with respect to the available energy to operate the respective columns or column setups.

The present disclosure has been described in conjunction with preferred embodiments and examples as well. However, other variations can be understood and effected by those persons skilled in the art and practicing the claimed invention, from the studies of the drawings, this disclosure and the claims.

Any steps presented herein can be performed in any order. The methods disclosed herein are not limited to a specific order of these steps. It is also not required that the different steps are performed at a certain place or in a certain computing node of a distributed system, i.e. each of the steps may be performed at different computing nodes using different equipment/data processing.

As used herein "determining" also includes "initiating or causing to determine", "generating" also includes "initiating and/or causing to generate" and "providing" also includes "initiating or causing to determine, generate, select, send and/or receive". "initiating or causing to perform an action" includes any processing signal that triggers a computing node or device to perform the respective action.

In the claims as well as in the description the word "comprising" or "including" or similar wording does not exclude other elements or steps and shall not be construed limiting to the elements or steps lined out. The indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation or further elements may be included. Providing in the scope of this disclosure may include any interface configured to provide data. This may include an application programming interface, a human-machine interface such as a display and/or a software module interface. Providing may include communication of data or submission of data to the interface, in particular display to a user or use of the data by the receiving entity. Providing may include retrieving, obtaining, receiving, gathering, or requesting.

Any disclosure and embodiments described herein relate to methods, systems, apparatuses, devices, chemicals, materials, services, uses, computer program elements lined out above and vice versa. Advantageously, the benefits provided by any of the embodiments and examples equally apply to all other embodiments and examples and vice versa.

All terms and definitions used herein are understood broadly and have their general meaning.

## Claims

1. A method for monitoring and/or controlling at least one thermal separation step in the production of aromatic amins and/or aromatic nitro chemicals, the method comprising the steps of:
- providing energy data associated with input energy supply characteristics and/or consumption characteristics,
- determining based on the energy data input data associated with one or more input mass stream(s) to be fed to the at least one thermal separation step,
- monitoring and/or controlling, based on the input data, the one or more input mass stream(s) fed to the at least one thermal separation step.

2. The method of claim 1, wherein the energy data relates to energy type and/or energy mix.

3. The method of claim 2, wherein the energy type relates to solar-, wind-, gas- or fossil-based energy, wherein the energy mix may relate to a mix of multiple energy types.

4. The method of any of the preceding claims, wherein energy data may relate to time dependent behavior of input energy supply characteristics and/or consumption characteristics

5. The method of any of the preceding claims, wherein the energy data relates to current input energy supply characteristics and/or consumption characteristics.

6. The method of any of the preceding claims, wherein input data is determined based on current input energy supply characteristics and/or consumption characteristics.

7. The method of any of the preceding claims, wherein determining input data includes determining quantity of input feed and/or heat input to be fed to the at least one thermal separation step.

8. The method of any of the preceding claims, wherein the input data is provided to a input feed for monitoring and/or controlling one or more input mass stream(s) fed to the at least one thermal separation step.

9. The method of any of the preceding claims, wherein depending on fluctuations in the energy data, different feed streams to at least one thermal separation step are adapted.

10. The method of any of the preceding claims, wherein the at least one thermal separation step includes a sequential column setup configured to separate ortho, -meta, para- nitrotoluol isomers.

11. The method of any of the preceding claims, wherein the at least one thermal separation step includes a sequential column setup configured to separate a toluidine mixture.

12. An apparatus for monitoring and/or controlling at least one thermal separation step in the production of aromatic amins and/or aromatic nitro chemicals, the apparatus configured to:
- providing energy data associated with input energy supply characteristics and/or consumption characteristics,
- determining based on the energy data input data associated with one or more input mass stream(s) to be fed to the at least one thermal separation step,
- monitoring and/or controlling, based on the input data, the one or more input mass stream(s) fed to the at least one thermal separation step.

13. Method for producing aromatic amins and aromatic nitro chemicals, the method comprising at least one thermal separation step, wherein the thermal separation step is monitored and/or controlled by the methods of any of claims 1-11 or apparatus of claim 12.

14. Use of the input data generated according to any of claims 1-11 for operating an industrial process.
